(19) Europäisches Patentamt
European Patent Office
Office européen des brevets

(11) EP 2 826 490 A1

(12) EUROPEAN PATENT APPLICATION
published in accordance with Art. 153(4) EPC

(43) Date of publication:
21.01.2015 Bulletin 2015/04

(21) Application number: 13761240.4

(22) Date of filing: 11.03.2013

(51) Int Cl.:
A61K 38/36 (2006.01)     A61K 36/00 (2006.01)
A61K 36/02 (2006.01)     A61P 31/04 (2006.01)
A61P 43/00 (2006.01)

(86) International application number:
PCT/JP2013/056652

(87) International publication number:
WO 2013/137197 (19.09.2013 Gazette 2013/38)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 13.03.2012 JP 2012055939

(71) Applicant: Fuji Chemical Co., Ltd.
Osaka-shi, Osaka 534-0024 (JP)

(72) Inventors:
• OHNO, Naoki
Nakatsugawa-shi
Gifu 509-9132 (JP)

• KAWANO, Akiko
Nakatsugawa-shi
Gifu 509-9132 (JP)
• INOUE, Takayasu
Nakatsugawa-shi
Gifu 509-9132 (JP)
• ISOBE, Hiroshi
Nakatsugawa-shi
Gifu 509-9132 (JP)

(74) Representative: Lahrtz, Fritz
Isenbruck Bösl Hörschler LLP
Patentanwälte
Prinzregentenstrasse 68
81675 München (DE)

(54) **BIOFILM INHIBITOR**

(57) The biofilm inhibitor of the present invention contains a lectin, at least one recognizable sugar chain of which is N-acetylglucosamine (G/LcNAc).

FIG. 3A

FIG. 3B

**Description**

CROSS-REFERENCE TO RELATED APPLICATIONS

This international application claims the priority based on Japanese Patent Application No. 2012-055939 filed March 13, 2012 in the Japan Patent Office, and the entire disclosure of Japanese Patent Application No. 2012-055939 is incorporated herein by reference.

TECHNICAL FIELD

The present invention relates to a biofilm inhibitor.

BACKGROUND ART

Biofilm is a structure formed with secretions or the like of one or more types of microorganisms that are gathered and adhered to the surface of various substances. Most microorganisms in the environment are present in the form of biofilm.
Biofilm is known to cause serious problems in human bodies, living environments, and medical fields.
In human bodies, biofilm can cause oral diseases such as dental caries, periodontal disease, oral malodor, etc. In addition, microbial infections in bodies as well as in oral cavities are said to be caused by microorganisms growing in biofilm. In the living environments, biofilm can be a cause of slime, clogging, and malodor in drains, pipes, etc. in a kitchen, and so on. In some cases, biofilm formed on the surface of equipment necessary for food processing causes food poisoning or the like, due to adhesion of microorganisms to food after processed. In the medical fields, biofilm formed on the surface of a catheter may cause serious infections. A number of methods to inhibit or remove biofilm have been reported. For example, Japanese Unexamined Patent Application Publication No. 2007-145876 reports that a composition for oral use incorporating sugar alcohol and/or amino acid therein suppresses adhesion, aggregation or the like of bacteria in the process of forming biofilm (Patent Document 1). Japanese Unexamined Patent Application Publication No. 2004-155681 reports that an oral biofilm inhibitor including a lactone derivative and/or a furan derivative that has at least one oxygen atom-containing functional group in a molecule and having a molecular weight of 220 or less, as an active ingredient, can effectively control oral bacteria and biofilm that cause oral diseases (Patent Document 2). Japanese Unexamined Patent Application Publication No. 2007-518400 reports that a cell-free fermentation product derived from one or more types of fermentative bacteria selected from Lactobacillus species, Lactococcus species, and Pediococcus species prevents biofilm formation, reduces existing biofilm, and decreases populations of bacteria (Patent Document 3).

PRIOR ART DOCUMENTS

PATENT DOCUMENTS

Patent Document 1: Japanese Unexamined Patent Application Publication No. 2007-145876
Patent Document 2: Japanese Unexamined Patent Application Publication No. 2004-155681
Patent Document 3: Japanese Unexamined Patent Application Publication No. 2007-518400

SUMMARY OF THE INVENTION

PROBLEMS TO BE SOLVED BY THE INVENTION

In the methods of removing or inhibiting a biofilm described in Patent Documents 1 to 3 described above, when the biofilm has a drug resistance, removal of microorganisms that form the biofilm becomes difficult. There is a problem in that a desired biofilm formation inhibitory effect cannot be achieved.
Therefore, in the present invention, it is desirable to provide a biofilm inhibitor excellent in biofilm formation inhibitory effect.

MEANS FOR SOLVING THE PROBLEMS

The biofilm inhibitor of the present invention is characterized in containing a lectin, at least one recognizable sugar chain of which is N-acetylglucosamine (GlcNAc).

By containing a lectin, at least one recognizable sugar chain of which is N-acetylglucosamine (GlcNAc), the biofilm inhibitor of the present invention has ability to inhibit a biofilm.

The reason why the effect of the present invention (have ability to inhibit a biofilm) is produced is estimated as described below.

A film of polysaccharide is formed on the surface of a biofilm. One of the components of the film of polysaccharide is N-acetylglucosamine (GlcNAc). As the lectin recognizes N-acetylglucosamine (GlcNAc) and binds to the film of polysaccharide, the film of polysaccharide is removed. Along with the removal of the film of polysaccharide, biofilm formation is inhibited. Thereby, it is possible to inhibit a biofilm.

In addition, the biofilm has acquired resistance to every drug by exchange of substances between the film of polysaccharide and microorganisms. According to the biofilm inhibitor of the present invention, since the film of polysaccharide is to be removed, it is possible to reduce resistance to drugs of the biofilm formed.

For the lectin, known lectins, at least one recognizable sugar chain of which is N-acetylglucosamine (GlcNAc), can be appropriately selected and used.

In addition, a lectin concentration in the biofilm inhibitor of the present invention is preferably 0.0001 mg/mL or above, more preferably 0.0001 mg/mL to 2 mg/mL, and further more preferably 0.0001 mg/mL to 1 mg/mL, in saturation concentration. The saturation concentration is the maximum concentration of lectin that can be contained in the biofilm inhibitor.

The biofilm inhibitor of the present invention preferably contains a lectin, at least one recognizable sugar chain of which is sialic acid (Sia).

If the biofilm inhibitor contains the lectin, at least one recognizable sugar chain of which is sialic acid (Sia), the lectin recognizes the sialic acid, and it is thereby possible to further inhibit a biofilm.

In this case, the reason why the effect of the present invention (to have ability to inhibit a biofilm) is produced is estimated as described below.

Sialic acid (Sia) is one of the components of the polysaccharide exposed on the surface of microorganisms. As the lectin recognizes the sialic acid (Sia) and binds to the microbial surface, the microorganisms firmly aggregate. Along with the firm aggregation of the microorganisms, adhesion to adherent bodies of the microorganisms is inhibited. Thus, it is possible to inhibit a biofilm.

In addition, the biofilm has acquired resistance to every drug by exchange of substances between the film of polysaccharide and microorganisms. According to the biofilm inhibitor of the present invention, the microorganisms firmly aggregate. Thus, it is possible to further reduce resistance to drugs of the biofilm formed.

As for the lectin, at least one recognizable sugar chain of which is sialic acid (Sia), known lectins may be appropriately selected and used.

In the biofilm inhibitor of the present invention, the lectin is preferably an extract from one or more natural products selected from the group consisting of bitter gourd, pokeweed, gorse, mushrooms, wheat, and jack bean.

In case that the lectin is an extract from one or more natural products selected from the group consisting of bitter gourd, pokeweed, gorse, mushrooms, wheat, and jack beans, the lectin recognizes N-acetylglucosamine (GlcNAc) and binds to the film of polysaccharide. Thereby, the film of polysaccharide is removed. Along with the removal of the film of polysaccharide, biofilm formation is inhibited. Thus, it is possible to further inhibit a biofilm. Further, in case that the lectin is an extract from wheat, mushrooms, or mushrooms and wheat, the lectin recognizes sialic acid (Sia) and binds to the microbial surface. Thereby, the microorganisms firmly aggregate. Along with the firm aggregation of microorganisms, adhesion to adherent bodies of microorganisms is inhibited. Thus, it is possible to further inhibit a biofilm.

In addition, the biofilm has acquired resistance to every drug by exchange of substances between the film of polysaccharide and microorganisms. According to the biofilm inhibitor of the present invention, since the film of polysaccharide is to be removed, it is possible to further lower the resistance to drugs of the biofilm formed. Further, in case that the lectin is an extract from mushrooms, wheat, or mushroom and wheat, the microorganisms firmly aggregate. Thus, it is possible to further reduce the resistance to drugs of the biofilm formed.

The lectin can be acquired by appropriately selecting and using known techniques from each of one or more natural products selected from the group consisting of bitter gourd, pokeweed, gorse, mushrooms, wheat, and jack bean. For example, it is possible to use the technique described below.

Each of the one or more natural products selected from the group consisting of bitter gourd, pokeweed, gorse, mushrooms, wheat, and jack bean is ground by a known method, using such as a coffee mill, a homogenizer, and the like, until ground to a desired size. The resulting powder is extracted using an extraction solution commonly used in extraction of protein (e.g., distilled water, Tris buffer, phosphate buffer, Tricine buffer, Hepes buffer, MOPS buffer, carbonate buffer, citrate buffer, borate buffer, MES buffer, and PIPES buffer; the extraction solution is not particularly limited to these). Ethanol is added to the acquired extract to acquire a precipitate. After the precipitate is dried, the dried precipitate is added to a physiological saline. Thereafter, the physiological saline is centrifuged to collect the supernatant. Thereby, it is possible to acquire a crude extract. The resulting crude extract is purified with an affinity column which binds sugar. Thereby, it is possible to acquire a lectin. Sugar to be bound to the affinity column is chosen depending on the sugar specificity of the lectin to be purified. For example, in the case of bitter gourd, the lectin can be purified efficiently with an affinity column which binds galactose. Further, if necessary, it is possible to add a technique such as gel filtration. Alternatively, or in addition, purification can be achieved using one or a combination of known techniques used for protein purification, for example, ammonium sulfate precipitation, sedimentation by organic solvent (ethanol, methanol, acetone, etc.), chromatography such as ion exchange chromatography, isoelectric chromatography, gel filtration chromatography, hydrophobic chromatography, adsorption chromatography, affinity chromatography using substrates or antibodies, and reverse phase column chromatography, filtration such as microfiltration, ultrafiltration, and reverse osmosis filtration.

Further, each lectin extracted from the one or more natural products selected from the group consisting of bitter gourd, pokeweed, gorse, mushrooms, wheat, and Jack bean, which are commercially available, may be appropriately selected and used.

In the biofilm inhibitor of the present invention, the lectin preferably has at least one of the following natures (1) and (2):

(1) nature to peel off at least one of the biofilm adhering to an adherent body and the bacteria forming the biofilm, from the adherent body; and
(2) nature to aggregate microorganisms that form the biofilm.

According to such biofilm inhibitor having at least one of the natures of (1) and (2), it possible to further inhibit a biofilm.

The adherent body can be any body as long as the biofilm can be adhered, such as, for example, teeth, dentures, dental plaque, drainage ditches of basin, bathroom, and toilet, or a catheter for medical use, and the like.

In the biofilm inhibitor of the present invention, the biofilm inhibitor preferably inhibits a biofilm formed by one or more microorganisms selected from the group consisting of Staphylococcus aureus, Candida albicans, Pseudomonas aeruginosa, and E. coli.

In the case of inhibiting a biofilm formed by one or more microorganisms selected from the group consisting of Staphylococcus aureus, Candida albicans, Pseudomonas aeruginosa, and E. coli, it is possible to suppress formation of fields where the microorganisms grow. Therefore, according to the biofilm inhibitor of the present invention, it is possible to eradicate these microorganisms and suppress infections caused by the microorganisms. Further, it is possible to suppress contamination of an adherent body caused by adhesion of these microorganisms.

BRIEF DESCRIPTION OF THE DRAWINGS

FIG. 1 is an explanatory diagram of a crystal violet method.
FIG. 2 is a picture showing results of observation of a stained condition of each well after step (c) of FIG. 1.
FIG. 3A is a graph showing the degree of biofilm formation, and FIG. 3B is a graph showing absorbance at a wavelength of 600 nm of each culture solution.
FIG. 4 is a picture showing results of observation of a stained condition of each well after step (c) of FIG. 1.
FIG. 5 is a graph showing the degree of biofilm formation.
FIG. 6 is a picture showing results of observation of a stained condition of each well after step (c) of FIG. 1.
FIG. 7A is a graph showing the degree of biofilm formation, and FIG. 7B is a diagram showing aggregation of Staphylococcus aureus.
FIG. 8 is a picture showing results of observation of a stained condition of each well after step (c) of FIG. 1.
FIG. 9 is a graph showing the degree of biofilm formation.
FIG. 10 is an explanatory diagram for explaining biofilm formation inhibitory effect of biofilm inhibitors 1 to 5 (in the figure, simply shown as inhibitors 1 to 5).
FIG. 11 is a picture showing results of observation of a stained condition of each well after step (c) of FIG. 1.
FIG. 12 is a graph showing the degree of biofilm formation.
FIG. 13 is a picture showing results of observation of a stained condition of each well after step (c) of FIG. 1.
FIG. 14 is a graph showing the degree of biofilm formation.
FIG. 15 is a picture showing results of observation of a stained condition of each well after step (c) of FIG. 1.
FIG. 16 is a graph showing the degree of biofilm formation.
FIG. 17 is a picture showing results of observation of a stained condition of each well after step (c) of FIG. 1.
FIG. 18 is a graph showing the degree of biofilm formation.
FIG. 19 is a picture showing results of observation of a stained condition of each well after step (c) of FIG. 1.
FIG. 20 is a graph showing the degree of biofilm formation.

MODE FOR CARRYING OUT THE INVENTION

Hereinafter, a description will be given, by way of example, for embodiments of the present invention. The present invention, however, should not to be limited to these embodiments. 1. Production of biofilm inhibitor

(Biofilm inhibitor 1 which contains bitter gourd lectin)

The biofilm inhibitor 1 which contains a bitter gourd lectin was produced as described below, based on the production methods described in Japanese Patent No. 3,849,945, and in Japanese Unexamined Patent Application Publication No. 2011-241192.

Bitter gourd seeds were pulverized into fine powder by a coffee mill. The fine powder acquired was placed in a container, to which distilled water was added, and the acquired solution was stirred with a stirrer. Thereafter, the solution was left for a while and seed components of bitter gourd were extracted. The extract was filtered, and the filtrate was centrifuged to collect the supernatant. Ethanol was added to the supernatant and stirred. The mixture was incubated for about 12 hours at 4°C, to acquire a bitter gourd crude extract.

The bitter gourd crude extract was purified with an affinity column which binds galactose, thereby to acquire a biofilm inhibitor 1.

(Biofilm inhibitor 2 which contains mushroom lectin)

A biofilm inhibitor 2 was prepared by adjusting the concentration of a mushroom lectin to a desired concentration. As the mushroom lectin, the model number J102 produced by Cosmo Bio Co., Ltd. was used.

(Biofilm inhibitor 3 which contains pokeweed lectin)

A biofilm inhibitor 2 was prepared by adjusting the concentration of a pokeweed lectin to a desired concentration. As the pokeweed lectin, the model number J116 produced by Cosmo Bio Co., Ltd. was used.

(Biofilm inhibitor 4 which contains wheat germ lectin)

A biofilm inhibitor 4 was prepared by adjusting the concentration of a wheat germ lectin to a desired concentration. As the wheat germ lectin, the model number J120 produced by Cosmo Bio Co., Ltd. was used.

(Biofilm inhibitor 5 which contains needle broom lectin)

A biofilm inhibitor 5 was prepared by adjusting the concentration of a furze lectin to a desired concentration. As the furze lectin, the model number J119 produced by Cosmo Bio Co., Ltd. was used.

(Biofilm inhibitor 6 which contains jack bean lectin)

A biofilm inhibitor 6 was prepared by adjusting the concentration of a jack bean lectin to a desired concentration. As the jack bean lectin, the model number J103 produced by Cosmo Bio Co., Ltd. was used.

(Comparative Example 1)

A biofilm inhibitor was prepared by adjusting the concentration of a soybean lectin to a desired concentration. As the soybean lectin, the model number J117 produced by Cosmo Bio Co., Ltd. was used.

(Comparative Example 2)

A biofilm inhibitor was prepared by adjusting the concentration of a pea lectin to a desired concentration. As the pea lectin, the model number J115 produced by Cosmo Bio Co., Ltd. was used.

(Comparative Example 3)

A biofilm inhibitor was prepared by adjusting the concentration of a lentil lectin to a desired concentration. As the lentil lectin, the model number J107 produced by Cosmo Bio Co., Ltd. was used.

(Comparative Example 4)

A biofilm inhibitor was prepared by adjusting the concentration of a peanut lectin to a desired concentration. As the peanut lectin, the model number J114 produced by Cosmo Bio Co., Ltd. was used.

(Comparative Example 5)

A biofilm inhibitor was prepared by adjusting the concentration of a lotus lectin to a desired concentration. As the lotus lectin, the model number J109 produced by Cosmo Bio Co., Ltd. was used.

(Comparative Example 6)

A biofilm inhibitor was prepared by adjusting the concentration of a Japanese elder lectin to a desired concentration. As the Japanese elder lectin, the model number J118 produced by Cosmo Bio Co., Ltd. was used.

2. Evaluation of biofilm inhibitor

(1) Evaluation of biofilm formation inhibitory effect in case that biofilm inhibitor 1 was added before formation of biofilm

(1-1) Evaluation method

Staphylococcus aureus was added to a glucose-added 804 medium and cultured until the optical density (opticaldensity: OD) of the medium at a wavelength of 600 nm is 0.1 OD. Thereafter, 500 $\mu$l of the culture solution was placed in each well of a 24-well multiwell plate. The biofilm inhibitor 1 was added to each well with the concentration of the bitter gourd lectin of 0.0001, 0.0005, 0.001, 0.005, 0.01, 0.05, 0.1, 0.5, and 1 mg/mL each. Thereafter, the culture solutions were cultured overnight. Also, a culture solution without addition of the biofilm inhibitor was cultured in the same manner as a control.
The composition of the glucose-added 804 medium is as shown below. Polypeptone 10 g/L, yeast extract 5 g/L, $MgSO_4 \cdot 7H_2O$ 1 g/L, glucose 5 g/L, distilled water 1 L
For each culture solution prepared, the degree of biofilm formation was calculated using a crystal violet method.

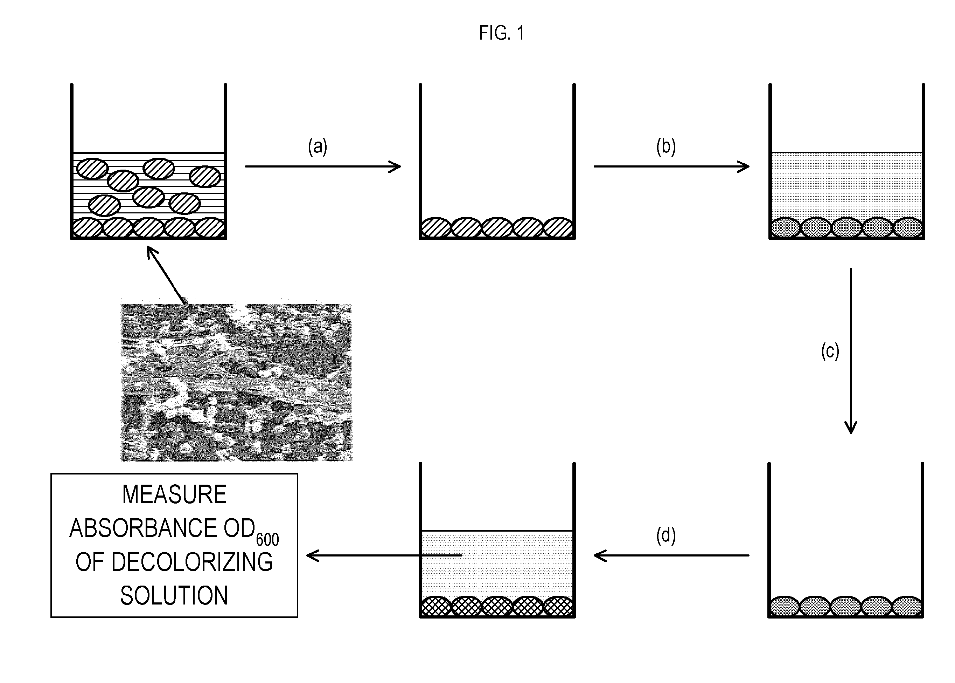

FIG. 1 shows an explanatory diagram that explains the crystal violet method. The culture solution was removed from each well and each well was washed with water and air-dried (step (a) in FIG. 1). After the step (a) in FIG. 1, what was remained fixed to each well was assumed a biofilm. Then, with addition of a crystal violet staining solution to each well, the biofilm was stained (step (b) in FIG. 1). Thereafter, the crystal violet staining solution was removed, and the biofilm was washed with water and air-dried (step (c) in FIG. 1). The stained condition of each well in this step was observed. Finally, with addition of ethanol to each well, the biofilm was discolored (step (d) in FIG. 1). The absorbance at a wavelength of 600 nm of the acquired decolorizing solution was measured. The degree of biofilm formation was calculated from the measured absorbance, using Equation 1. Equation 1 is as shown below:

$$(\text{Equation 1}) \; \text{Degree of biofilm formation (\%)} = X/Y \times 100$$

where X is the absorbance for the well to which the biofilm inhibitor was added, which was acquired by measuring the decolorizing solution after the step (d) in FIG. 1, and Y is the absorbance for the control well, which was acquired by measuring the decolorizing solution after the step (d) in FIG. 1.

(1-2) Results

[Results for staining each well after step (c) in FIG. 1]

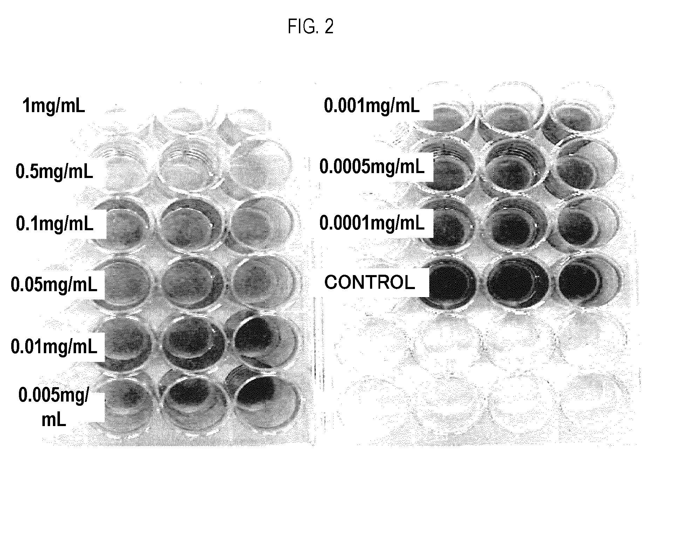

FIG. 2 shows results of observation of the stained condition of each well after the step (c) in FIG. 1 above. As shown in FIG. 2, it was found that, as the concentration of the bitter gourd lectin becomes higher from 0.0001 to 0.0005, 0.001, 0.005, 0.01, 0.05, 0.1, 0.5, and 1 mg/mL, the degree of staining becomes lower than that of the control. It was possible to visually recognize that the biofilm formation inhibitory effect is high.

[Results of calculating degree of biofilm formation]

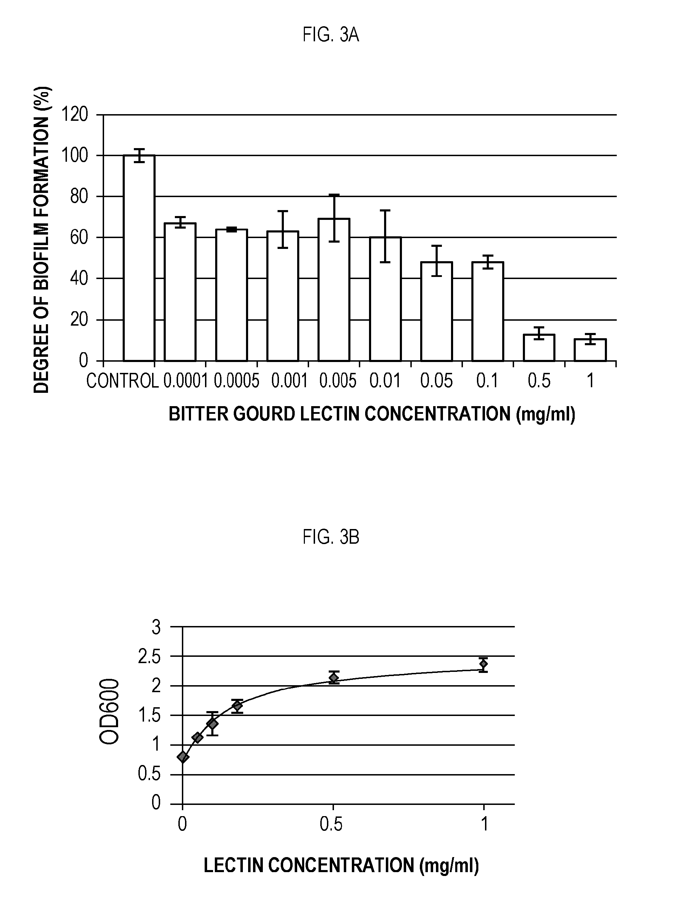

FIG. 3A shows results of calculating the degree of biofilm formation. In case that the bitter gourd lectin was added with the concentration of 0.0001, 0.0005, 0.001, 0.005, and 0.01 mg/mL, the degree of biofilm formation was about 60%. It was found that the biofilm formation inhibitory effect is high as compared with that of the control. Further, the degree of biofilm formation was further reduced at the concentration of 0.05 mg/mL or above. It was shown that the biofilm formation inhibitory effect has been improved.

FIG. 3B shows results of measurement of the absorbance (OD600) at a wavelength of 600 nm in the culture solutions after cultured overnight, to which the bitter gourd lectin was added with the concentration of 0.01, 0.05, 0.1, 0.5, and 1 mg/mL.

As shown in FIG. 3B, it was found that, as the concentration of the bitter gourd lectin rises, the absorbance increases, and the number of airborne bacteria of Staphylococcus aureus increases in a power function manner. From the results, it was suggested that the biofilm inhibitor 1 produces the biofilm formation inhibitory effect as a result that the bitter gourd lectin suppresses adhesion of Staphylococcus aureus to the plate or peels off the adhered Staphylococcus aureus.

(2) Evaluation of biofilm formation inhibitory effect in case that each of biofilm inhibitor 1, biofilm inhibitor 5, and biofilm inhibitors of Comparative Examples 1 to 3 was added before formation of biofilm

(1-1) Evaluation method

Evaluation was made in the same manner as in the evaluation method described in (1-1) of (1) above, except that each of the biofilm inhibitor 1, the biofilm inhibitor 5, and the biofilm inhibitors of Comparative Examples 1 to 3, of which lectin concentration is 0.5 mg/mL, was used as a biofilm inhibitor.

(1-2) Results

[Results for staining each well after step (c) in FIG. 1]

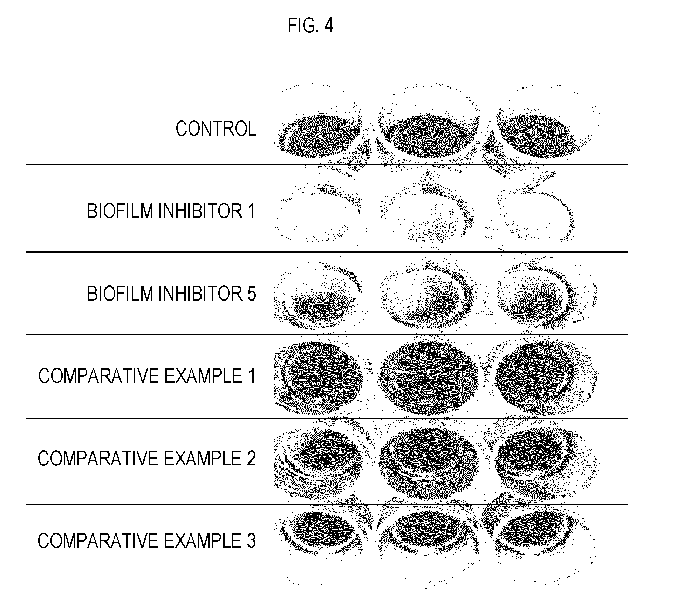

FIG. 4 shows results of observation of the stained condition of each well after step (c) in FIG. 1. As shown in FIG. 4, it was shown that the biofilm inhibitor 1 and the biofilm inhibitor 5 have low staining intensity as compared with the control and the biofilm inhibitors of Comparative Examples 1 to 3. It was possible to visually confirm that the biofilm formation inhibitory effect is high. Furthermore, it was shown that the staining intensity is the lowest when the biofilm inhibitor 1 is used, and the staining intensity is the second lowest when the biofilm inhibitor 2 is used.

[Results of calculating degree of biofilm formation]

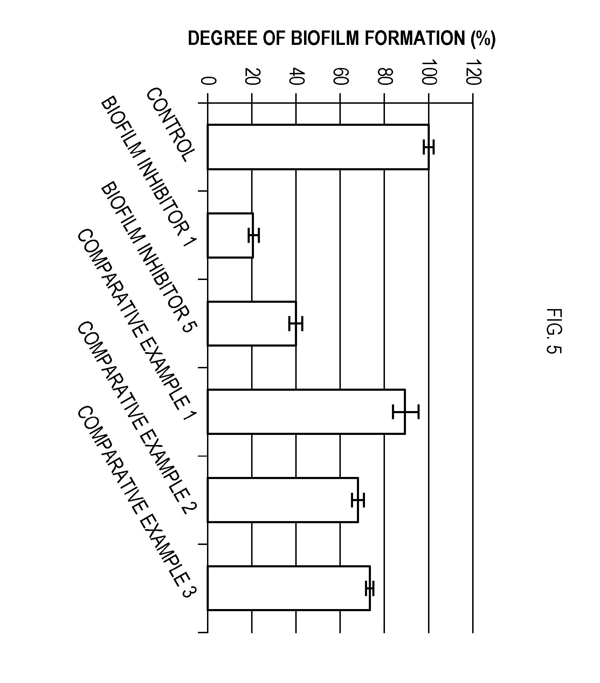

FIG. 5 shows results of calculating the degree of biofilm formation in the case of using each biofilm inhibitor.

As shown in FIG. 5, the degree of biofilm formation of the biofilm inhibitor 1 was about 20%, and the degree of biofilm formation of the biofilm inhibitor 5 was about 40%. It was shown that the biofilm inhibitor 1 and the biofilm inhibitor 5 have significantly high biofilm formation inhibitory effect as compared with the control and the biofilm inhibitors of Comparative Examples 1-3.

(3) Evaluation of biofilm formation inhibitory effect in case that each of biofilm inhibitor 1 and biofilm inhibitor 2 was added before formation of biofilm

(1-1) Evaluation method

Evaluation was performed in the same manner as in the evaluation method described in (1-1) of (1) above, except that each of the biofilm inhibitor 1 of which lectin concentration is 0.5 mg/mL and the biofilm inhibitor 2 of which lectin concentration is 0.5 mg/mL is used as a biofilm inhibitor.

(1-2) Results

[Results for staining each well after step (c) in FIG. 1]

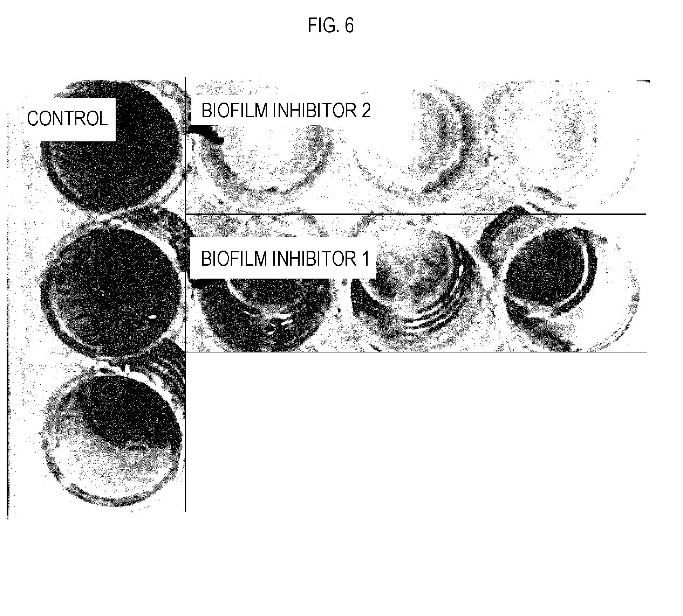

FIG. 6 shows results of observation of the stained condition of each well after the step (c) in FIG. 1. As shown in FIG. 6, the biofilm inhibitor 1 and the biofilm inhibitor 2 had low staining intensity as compared with the control. It was possible to visually confirm that the biofilm inhibitor 1 and the biofilm inhibitor 2 have high biofilm formation inhibitory effect.

[Results of calculating degree of biofilm formation]

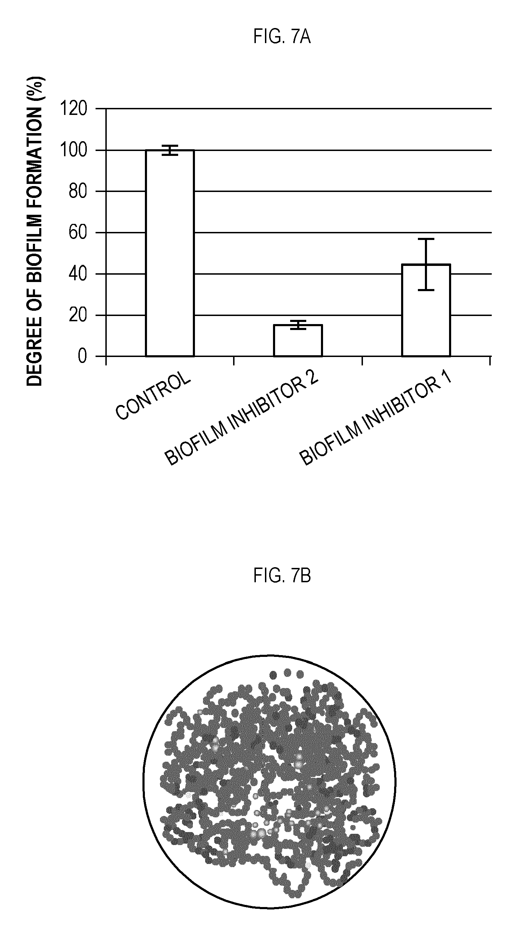

FIG. 7A shows results of calculating the degree of biofilm formation in the case of using each biofilm inhibitor.
As shown in FIG. 7A, it was seen that the biofilm inhibitor 1 and the biofilm inhibitor 2 have significantly reduced degree of biofilm formation as compared with the control, and thus have high biofilm formation inhibitory effect.
As shown in FIG. 7B, in case that the biofilm inhibitor 2 is used, aggregation of Staphylococcus aureus was observed immediately after the biofilm inhibitor 2 was added. In case that the biofilm inhibitor 1 was added, aggregation of Staphylococcus aureus was not observed. (4) Evaluation of biofilm formation inhibitory effect in case that each of biofilm inhibitor 1, biofilm inhibitor 3, biofilm inhibitor 4, and biofilm inhibitors of Comparative Examples 4 to 6 was added before formation of bio film

(1-1) Evaluation method

Evaluation was performed in the same manner as in the evaluation method described in (1-1) of (1) above, except that each of the biofilm inhibitor 1, the biofilm inhibitor 3, the biofilm inhibitor 4, and the biofilm inhibitors of Comparative Examples 4 to 6, of which lectin concentration is 0.5 mg/mL, is used as a biofilm inhibitor.

(1-2) Results

[Results for staining each well after step (c) in FIG. 1]

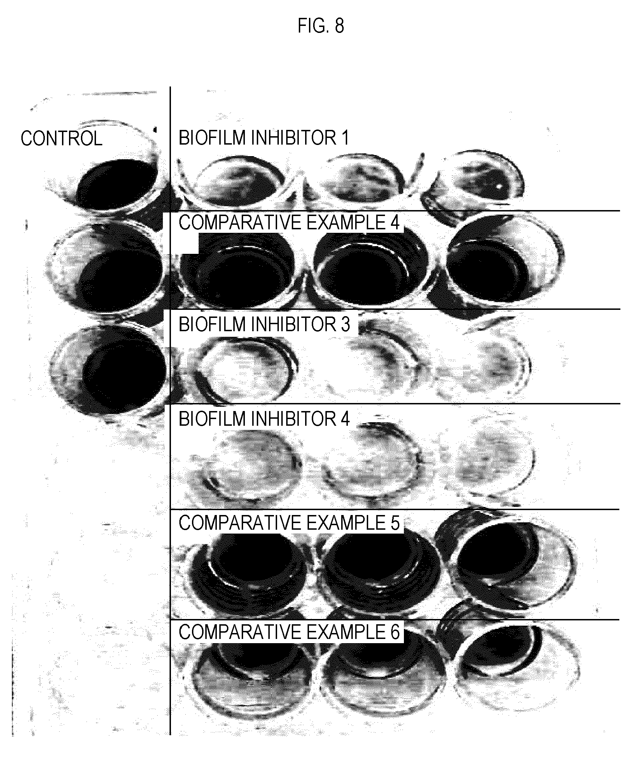

FIG. 8 shows results of observation of the stained condition of each well after the step (c) in FIG. 1.
As shown in FIG. 8, it was seen that the biofilm inhibitor 1, the biofilm inhibitor 3, and the biofilm inhibitor 4 have remarkably lowered staining intensity as compared with the control and the biofilm inhibitors of Comparative Examples 4 to 6. It was possible to visually confirm that the biofilm inhibitor 1, the biofilm inhibitor 3, and the biofilm inhibitor 4 have high biofilm formation inhibitory effect.

[Results of calculating degree of biofilm formation]

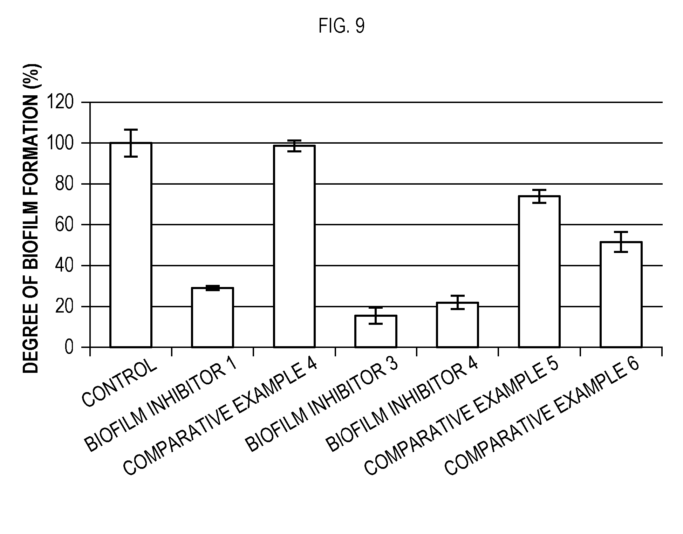

FIG. 9 shows results of calculating the degree of biofilm formation in the case of using each biofilm inhibitor.
As shown in FIG. 9, the biofilm inhibitor 1, the biofilm inhibitor 3, and the biofilm inhibitor 4 have significantly reduced degree of biofilm formation as compared with the control and the biofilm inhibitors of Comparative Examples 4 to 6. It was found that the biofilm inhibitor 1, the biofilm inhibitor 3, and the biofilm inhibitor 4 have high biofilm formation inhibitory effect.
In case that the biofilm inhibitor 4 is added, aggregation of Staphylococcus aureus was observed as in the case that the biofilm inhibitor 2 is added.
Table 1 summarizes the above results. The higher the number of "+" is, the higher the biofilm formation inhibitory effect is. The same number of "+" indicates that the biofilm formation inhibitory effect is equivalent. In addition, sugar chains recognized by the lectin contained in each biofilm inhibitor are also shown in Table 1. It is to be noted that effect on Pseudomonas aeruginosa is shown, in regard to the biofilm inhibitor 6.

[Table 1]

| Lectin Name | Recognition Sugar and Sugar Chain | Biofilm Formation Inhibitory Effect |
|---|---|---|
| Biofilm Inhibitor 1 | D-Gal/GalNAc<br>Fuc$\alpha$1-2Gal$\beta$1-4GlcNAc | +++ (peeling effect) |
| Biofilm Inhibitor 2 | $\beta$-D-Gal, GlcNAc<br>Gal$\beta$1-3GalNAc$\alpha$1-Ser/Thr<br>Sia$\alpha$2-3($\beta$)Gal$\beta$1-3GalNAc $\alpha$1-Ser/Thr | +++ (aggregation effect) |
| Biofilm Inhibitor 3 | [$\beta$1-4GlcNAc]n | +++ (peeling effect) |
| Biofilm Inhibitor 4 | D-GlcNAc, Sia<br>GlcNAc$\beta$1-4GlcNAc$\beta$1-4GlcNAc<br>GlcNAc$\beta$1-4GlcNAc | +++ (aggregation effect) |
| Biofilm Inhibitor 5 | $\alpha$-L-Fuc<br>Fuc$\alpha$1-2Gal$\beta$1-4GlcNAc | +++ (peeling effect) |

(continued)

| Lectin Name | Recognition Sugar and Sugar Chain | Biofilm Formation Inhibitory Effect |
|---|---|---|
| Biofilm Inhibitor 6 | α-D-Man, α-D-Glc<br>High mannose type(N-linked)<br>Galβ1-4GlcNAcβ1-2/4Man | +++ (peeling effect on Pseudomonas aeruginosa) |
| Comparative Example 1 | D-GalNAc<br>GalNAcα1-3Gal of O-linked glycopeptides | - |
| Comparative Example 2 | D-Man, D-Glc, L-Fuc | + |
| Comparative Example 3 | D-Man, D-Glc, L-Fuc | + |
| Comparative Example 4 | D-Gal<br>Galβ1-3GalNAc of (7-linked glycopeptides | - |
| Comparative Example 5 | L-Fuc<br>Lewis Y | + |
| Comparative Example 6 | Siaα2-βGal/GalNAc | ++ |
| -: No Biofilm Formation Inhibitory Effect Gal: galactose<br>GalNAc: N-acetylgalactosamine<br>Glc: glucose<br>GlcNAc: N-acetylglucosamine<br>Fuc: fucose<br>Man: mannose<br>Sia: sialic acid<br>Ser/Thr: serine/threonine | | |

As shown in Table 1, the biofilm inhibitors 1 to 6 containing a lectin including N-acetylglucosamine (GlcNAc) in the recognizable sugar chain can achieve high biofilm formation inhibitory effect. From the results, it was shown that the lectin including N-acetylglucosamine (GlcNAc) in the recognizable sugar chain can inhibit formation of biofilm.

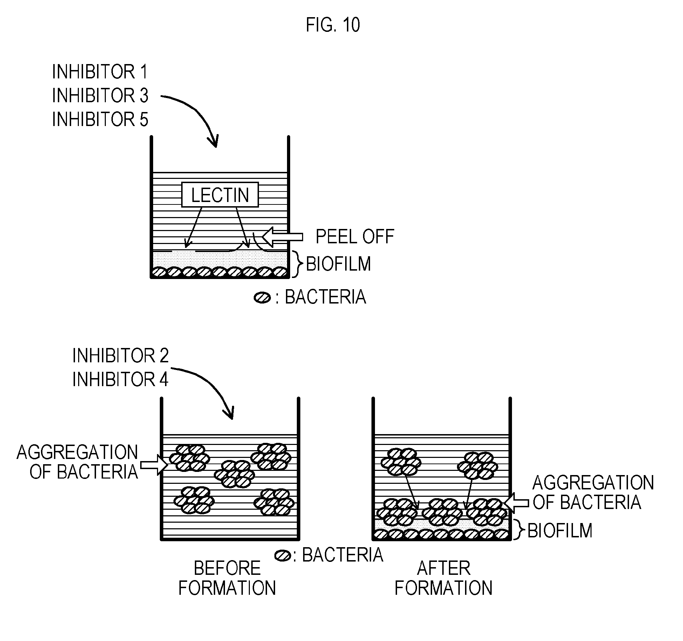

Further, the biofilm inhibitor 2 and the biofilm inhibitor 4 can firmly aggregate bacteria and inhibit formation of biofilm. Since the biofilm inhibitor 2 and the biofilm inhibitor 4 contain a lectin including sialic acid (Sia) as well as N-acetylglucosamine (GlcNAc) in the recognizable sugar chain, it was shown that the lectin containing sialic acid (Sia) as well as N-acetylglucosamine (GlcNAc) in the recognizable sugar chain can firmly aggregate bacteria and inhibit formation of biofilm (see FIG. 10). In FIG 10, the "biofilm inhibitor" is simply described as "inhibitor", and "before formation (or after formation) of biofilm" is simply described as "before formation (or after formation)".

(5) Evaluation of peeling effect of biofilm in case that each of biofilm inhibitor 1 and biofilm inhibitor 2 was added after formation of biofilm

(1-1) Evaluation method

Staphylococcus aureus was added to a glucose-added 804 medium and cultured until the optical density (opticaldensity: OD) of the medium at a wavelength of 600 nm is 0.1 OD. Thereafter, 500 μl of the culture solution was placed in each well of a 24-well multiwell plate. The culture solution was cultured overnight, to form a biofilm in each well. Then, to each well, each of the biofilm inhibitor 1 of which lectin concentration is 0.5 mg/mL and the biofilm inhibitor 2 of which lectin concentration is 0.5 mg/mL was added.

The degree of biofilm formation was calculated in the same manner as described in (1-1) of (1) above.

(1-2) Results

[Results for staining each well after step (c) in FIG. 1]

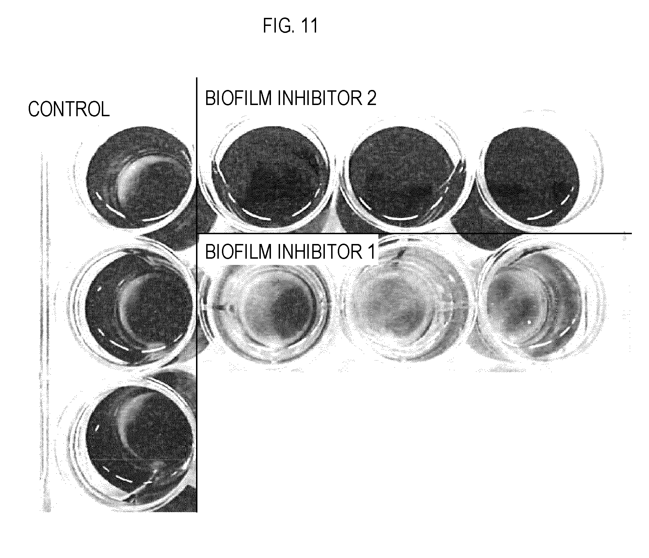

FIG. 11 shows results of observation of the stained condition of each well after the step (c) in FIG. 1.
As shown in FIG. 11, the biofilm inhibitor 1 has low staining intensity as compared with the control. This is due to the fact that a formed biofilm that is adhered to each well was peeled off. Accordingly, it was possible to visually confirm that the biofilm inhibitor 1 has high biofilm peeling effect. The staining intensity of the biofilm inhibitor 2 increased as compared with that of the control. It was visually observed that adhesion of biofilm is strengthened.

[Results of calculating degree of biofilm formation]

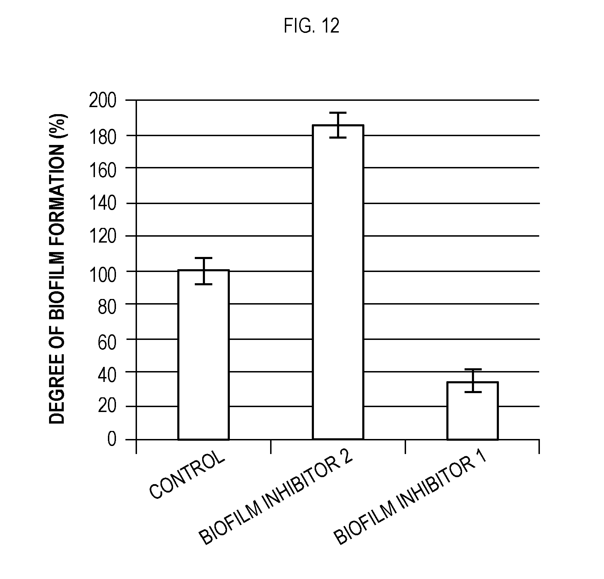

FIG. 12 shows results of calculating the degree of biofilm formation in the case of using each biofilm inhibitor.
As shown in FIG. 12, in case that the biofilm inhibitor 1 is used, the degree of biofilm formation is significantly reduced. It was found that the biofilm inhibitor 1 has high biofilm peeling effect, as compared with the control. Further, since the degree of biofilm formation increases about twofold when the biofilm inhibitor 2 is used, it was found that the biofilm inhibitor 2 has low biofilm peeling effect, as compared with the control. (6) Evaluation of biofilm peeling effect in case that each of biofilm inhibitor 1, biofilm inhibitor 3 and biofilm inhibitor 4 was added after formation of biofilm

(1-1) Evaluation method

Evaluation was performed in the same manner as the evaluation method described in (1-1) of (5) above, except that each of the biofilm inhibitor 1 of which lectin concentration is 0.5 mg/mL, the biofilm inhibitor 3 of which lectin concentration is 0.5 mg/mL and the biofilm inhibitor 4 of which lectin concentration is 0.5 mg/mL is used.
(1-2) Results

[Results for staining each well after step (c) in FIG. 1]

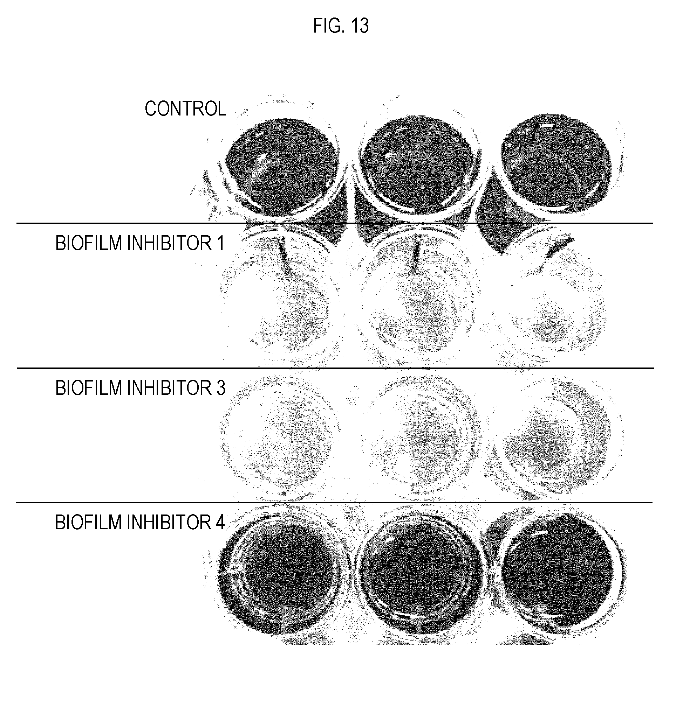

FIG. 13 shows results of observation of the stained condition of each well after the step (c) in FIG. 1.
As shown in FIG. 13, the biofilm inhibitor 1 and the biofilm inhibitor 3 had low staining intensity as compared with the control. Accordingly, it was possible to visually confirm that the biofilm inhibitor 1 and the biofilm inhibitor 3 have high biofilm peeling effect. Since the staining intensity increased as compared with that of the control when the biofilm inhibitor 4 is used, it was visually observed that adhesion of biofilm is enhanced.

[Result of calculating degree of biofilm formation]

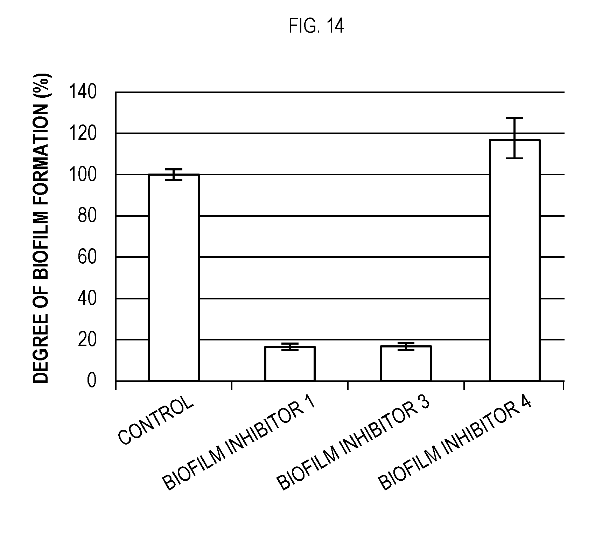

FIG. 14 shows results of calculating the degree of biofilm formation in the case of using each biofilm inhibitor.
As shown in FIG. 14, in case that the biofilm inhibitor 1 and the biofilm inhibitor 3 are used, the degree of biofilm formation is reduced significantly. It was found that the biofilm inhibitor 1 and the biofilm inhibitor 3 have high biofilm peeling effect as compared with the control. Also, in case that the biofilm inhibitor 4 is used, since the degree of biofilm formation increases as compared with that of the control, it was found that the biofilm peeling effect is low.
Table 1 summarizes results of the above.
The biofilm inhibitor 1, the biofilm inhibitor 3 and the biofilm inhibitor 5 can produce high peeling effect on a biofilm formed. Since the biofilm inhibitor 1 and the biofilm inhibitor 5 contain a lectin including L-fucose-D-galactose-N-acetylglucosamine (Fuc$\alpha$1-2Gal$\beta$1-4GlcNAc) in the recognizable sugar chain, it was suggested that the lectin including L-fucose-D-galactose-N-acetylglucosamine (Fuc$\alpha$1-2Gal$\beta$1-4GlcNAc) in the recognizable sugar chain can peel biofilm. Further, since the biofilm inhibitor 3 contains a lectin that recognizes N-acetylglucosamine (linked ($\beta$1-4GlcNAc)n), it was suggested that the lectin that recognizes N-acetylglucosamine (linked ($\beta$1-4GlcNAc)n) has ability to peel biofilm (see
FIG. 10).

(7) Evaluation of biofilm formation inhibitory effect in case that each of biofilm inhibitor 1 and biofilm inhibitor 3 was added to biofilm formed by Candida albicans before formation of biofilm

(1-1) Evaluation method

Evaluation was performed in the same manner as described in (1-1) of (1) above, except that candida is used as a bacteria to form a biofilm, and each of the biofilm inhibitor 1 of which lectin concentration is 0.5 mg/mL and the biofilm inhibitor 3 of which lectin concentration is 0.5 mg/mL is used as a biofilm inhibitor.

(1-2) Results

[Results for staining each well after step (c) in FIG. 1]

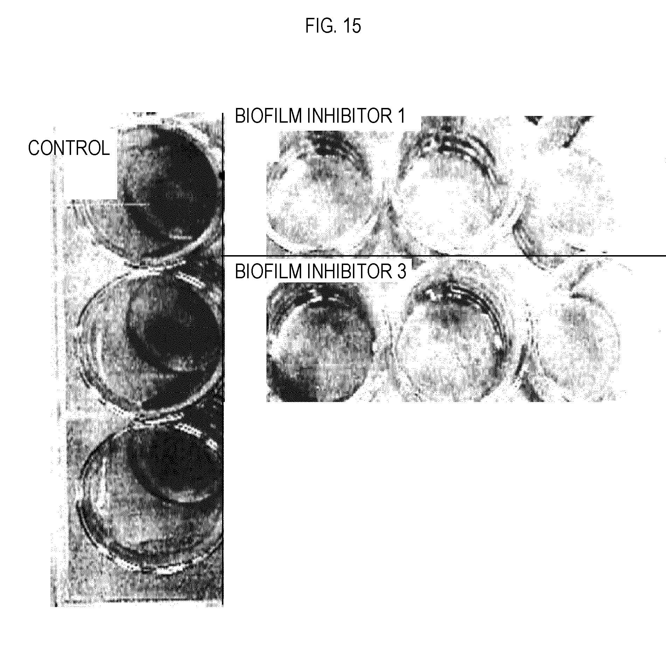

FIG. 15 shows results of observation of the stained condition of each well after step (c) in FIG. 1.
As shown in FIG. 15, since the biofilm inhibitor 1 and the biofilm inhibitor 3 had low staining intensity as compared with the control, it was possible to visually confirm that the biofilm inhibitor 1 and the biofilm inhibitor 3 have high biofilm formation inhibitory effect.

[Results of calculating degree of biofilm formation]

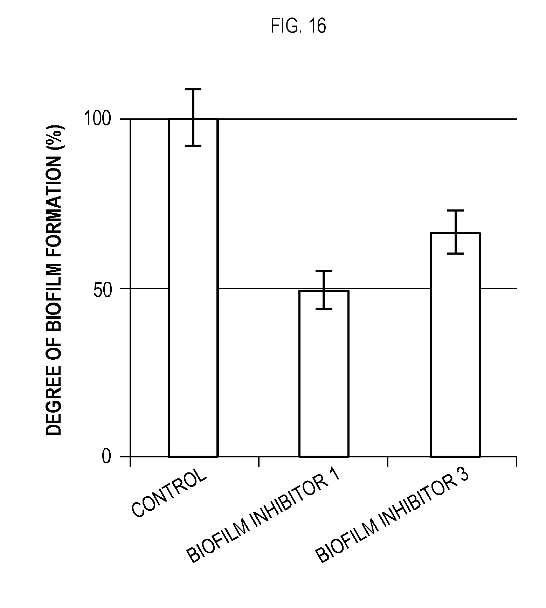

FIG. 16 shows results of calculating the degree of biofilm formation in the case of using each biofilm inhibitor.
As shown in FIG. 16, since the degree of biofilm formation in the biofilm inhibitor 1 and the biofilm inhibitor 3 is lowered as compared with that of the control, it was found that the biofilm inhibitor 1 and the biofilm inhibitor 3 have high biofilm formation inhibitory effect.

(8) Evaluation of biofilm formation inhibitory effect in case that each of biofilm inhibitor 3, biofilm inhibitor 4, and biofilm inhibitor 6 was added to biofilm formed by Pseudomonas aeruginosa before formation of biofilm

(1-1) Evaluation method

Evaluation was performed in the same manner as described in (1-1) of (1) above, except that Pseudomonas aeruginosa is used as a bacteria to form a biofilm and each of the biofilm inhibitor 3, the biofilm inhibitor 4, and the biofilm inhibitor 6, of which lectin concentration is 0.5 mg/mL, is used for a biofilm inhibitor.

(1-2) Results

[Results for staining each well after step (c) in FIG. 1]

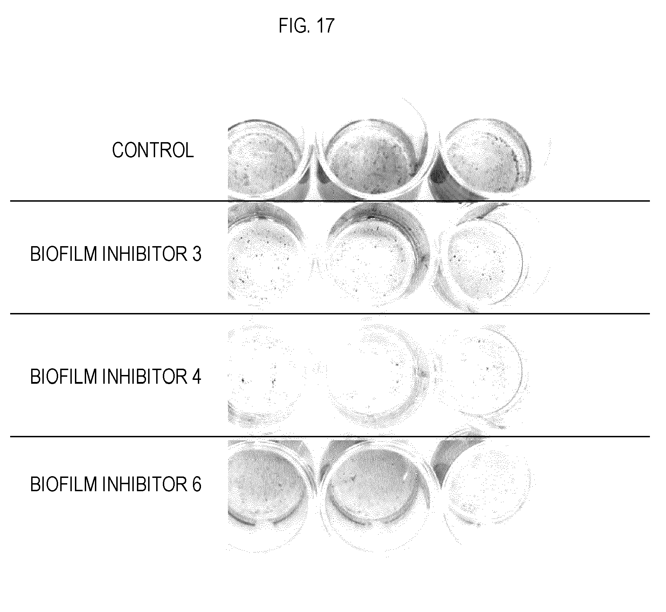

FIG. 17 shows results of observation of the stained condition of each well after the step (c) in FIG. 1.
As shown in FIG. 17, it was seen that the biofilm inhibitor 3, the biofilm inhibitor 4, and the biofilm inhibitor 6 have low staining intensity as compared with the control. It was possible to visually confirm that the biofilm inhibitor 3, the biofilm inhibitor 4, and the biofilm inhibitor 6 have high biofilm formation inhibitory effect.

[Results of calculating degree of biofilm formation]

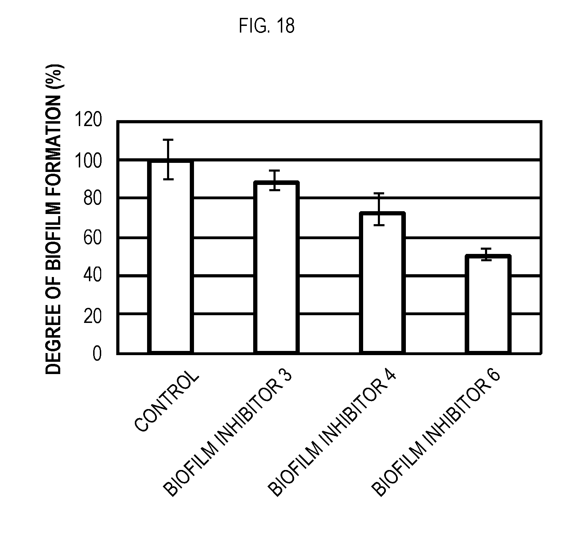

FIG. 18 shows results of calculating the degree of biofilm formation in the case of using each biofilm inhibitor.
As shown in FIG. 18, since the degree of biofilm formation in the biofilm inhibitor 3, the biofilm inhibitor 4, and the biofilm inhibitor 6 was lowered as compared with that of the control, it was found that the biofilm inhibitor 3, the biofilm inhibitor 4, and the biofilm inhibitor 6 have high biofilm formation inhibitory effect.

(9) Evaluation of biofilm formation inhibitory effect in case that biofilm inhibitor 1 or biofilm inhibitor 4 was added to biofilm formed by Escherichia coli before formation of biofilm

(1-1) Evaluation method

Evaluation was performed in the same manner as described in (1-1) of (1) above, except that E. coli is used as a bacteria to form a biofilm and each of the biofilm inhibitor 1 and the biofilm inhibitor 4, of which lectin concentration is 0.5 mg/mL, is used as a biofilm inhibitor.

(1-2) Results

[Results for staining each well after step (c) in FIG. 1]

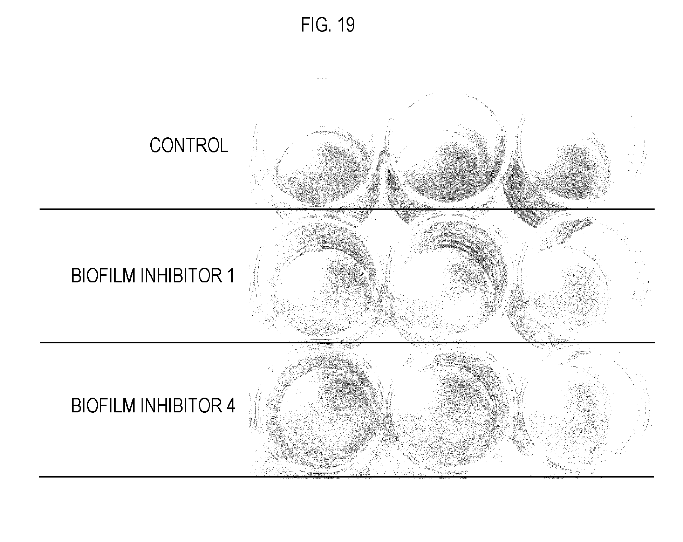

FIG. 19 shows results of observing the stained condition of each well after step (c) in FIG. 1.
As shown in FIG. 19, it was seen that the staining intensity in the biofilm inhibitor 1 and the biofilm inhibitor 4 is low as compared with the control. It was possible to visually confirm that the biofilm inhibitor 1 and the biofilm inhibitor 4 have high biofilm formation inhibitory effect.

[Results of calculating degree of biofilm formation]

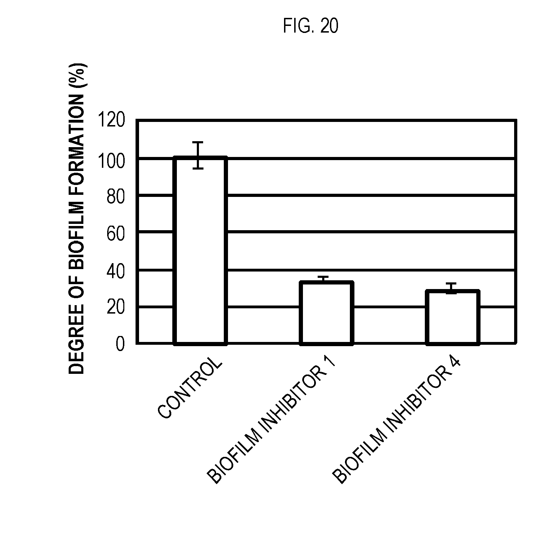

FIG. 20 shows results of calculating the degree of biofilm formation in the case of using each biofilm inhibitor.
As shown in FIG. 20, it was found that the biofilm inhibitor 1 and the biofilm inhibitor 4 have high biofilm formation inhibitory effect, since the degree of biofilm formation was lowered as compared with that of the control.
The present invention is not limited to the embodiments described above and can be practiced in various modes within a scope not departing from the present invention.

## Claims

1. A biofilm inhibitor comprising a lectin at least one recognizable sugar chain of which is N-acetyl glucosamine.

2. The biofilm inhibitor according to claim 1, comprising a lectin at least one recognizable sugar chain of which is sialic acid (Sia).

3. The biofilm inhibitor according to claim 1, wherein the lectin is an extract from one or more types of natural products selected from the group consisting of bitter gourd, pokeweed, gorse, mushrooms, wheat, and jack bean.

4. The biofilm inhibitor according to claim 2, wherein the lectin is an extract from mushroom, wheat, or mushroom and wheat.

5. The biofilm inhibitor according to one of claims 1 to 4, wherein the lectin has a nature of at least one of the following (1) and (2):

   (1) nature that peels off at least one of biofilms adhering to an adherent body and bacteria to form biofilms from the adherent body; and
   (2) nature to aggregate microorganisms that form biofilms.

6. The biofilm inhibitor according to one of claims 1 to 5, wherein the biofilm inhibitor inhibits a biofilm formed by one or more of microorganisms selected from the group consisting of Staphylococcus aureus, Candida albicans, Pseudomonas aeruginosa, and E. coli.

FIG. 1

MEASURE
ABSORBANCE $OD_{600}$
OF DECOLORIZING
SOLUTION

FIG. 2

FIG. 3A

FIG. 3B

FIG. 4

CONTROL

BIOFILM INHIBITOR 1

BIOFILM INHIBITOR 5

COMPARATIVE EXAMPLE 1

COMPARATIVE EXAMPLE 2

COMPARATIVE EXAMPLE 3

FIG. 5

FIG. 6

FIG. 7A

FIG. 7B

FIG. 8

FIG. 9

FIG. 10

INHIBITOR 1
INHIBITOR 3
INHIBITOR 5

LECTIN

PEEL OFF

}BIOFILM

⌀ : BACTERIA

INHIBITOR 2
INHIBITOR 4

AGGREGATION
OF BACTERIA

AGGREGATION
OF BACTERIA

}BIOFILM

⌀: BACTERIA

BEFORE
FORMATION

AFTER
FORMATION

FIG. 11

CONTROL

BIOFILM INHIBITOR 2

BIOFILM INHIBITOR 1

FIG. 12

FIG. 13

CONTROL

BIOFILM INHIBITOR 1

BIOFILM INHIBITOR 3

BIOFILM INHIBITOR 4

FIG. 14

EP 2 826 490 A1

FIG. 15

CONTROL

BIOFILM INHIBITOR 1

BIOFILM INHIBITOR 3

FIG. 16

FIG. 17

CONTROL

BIOFILM INHIBITOR 3

BIOFILM INHIBITOR 4

BIOFILM INHIBITOR 6

FIG. 18

FIG. 19

CONTROL

BIOFILM INHIBITOR 1

BIOFILM INHIBITOR 4

FIG. 20

## INTERNATIONAL SEARCH REPORT

| International application No. |
|---|
| PCT/JP2013/056652 |

A. CLASSIFICATION OF SUBJECT MATTER
*A61K38/36*(2006.01)i, *A61K36/00*(2006.01)i, *A61K36/02*(2006.01)i, *A61P31/04*
(2006.01)i, *A61P43/00*(2006.01)i

According to International Patent Classification (IPC) or to both national classification and IPC

B. FIELDS SEARCHED

Minimum documentation searched (classification system followed by classification symbols)
A61K38/36, A61K36/00, A61K36/02, A61P31/04, A61P43/00

Documentation searched other than minimum documentation to the extent that such documents are included in the fields searched
Jitsuyo Shinan Koho          1922-1996   Jitsuyo Shinan Toroku Koho   1996-2013
Kokai Jitsuyo Shinan Koho    1971-2013   Toroku Jitsuyo Shinan Koho   1994-2013

Electronic data base consulted during the international search (name of data base and, where practicable, search terms used)
CAplus(STN), BIOSIS(STN), EMBASE(STN), MEDLINE(STN),
JSTPlus/JMEDPlus/JST7580(JDreamII), JSTPatM

C. DOCUMENTS CONSIDERED TO BE RELEVANT

| Category* | Citation of document, with indication, where appropriate, of the relevant passages | Relevant to claim No. |
|---|---|---|
| X<br>Y | Takashi ITO et al., "Endai B3 (Shishu) [3301]<br>'Streptococcus mutans no Biofilm Keisei ni<br>Taisuru Lectin ni yoru Yokusei Kiko'", Program<br>and Abstracts, The 133rd Meeting of the<br>Japanese Society of Conservative Dentistry<br>2010 Nendo Shuki Gakujutsu Taikai, The 12th<br>JSCD/KACD Joint-Scientific Meeting, 20 October<br>2010 (20.10.2010), page 40, entire text | 1-3,5<br>3,4 |
| X<br>Y | JP 2011-246465 A  (Okayama University),<br>08 December 2011 (08.12.2011),<br>claims 1, 6; paragraphs [0001], [0002]; example<br>3; fig. 2<br>(Family: none) | 1-3,5<br>3,4 |

| ☒ | Further documents are listed in the continuation of Box C. | | ☐ | See patent family annex. |
|---|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 27 March, 2013 (27.03.13) | 09 April, 2013 (09.04.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/056652 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y | WO 2010/050369 A1 (Okayama University),<br>06 May 2010 (06.05.2010),<br>claims 1, 4, 7; paragraphs [0002], [0003],<br>[0035]; example 3; table 2; fig. 8<br>(Family: none) | 1-3,5<br>3,4 |
| Y | JP 2011-15640 A (University of Miyazaki),<br>27 January 2011 (27.01.2011),<br>paragraph [0099]; table 2<br>(Family: none) | 3 |
| Y | JP 2009-209072 A (National University<br>Corporation Shizuoka University),<br>17 September 2009 (17.09.2009),<br>paragraph [0016]<br>(Family: none) | 3 |
| Y | 'Lectin Tokuisei Ichiranhyo', Funakoshi ·<br>Toshitsu Kenkyuyo Seihin [Sanko Shiryo],<br>[online], 11 August 2008 (11.08.2008),<br>Funakoshi Co., Ltd., [retrieved on 26 March<br>2013 (26.03.2013)], Internet <URL http://www.<br>funakoshi.co.jp/contents/3078>, list | 3,4 |
| Y | JP 2011-502714 A (Medtronic MiniMed, Inc.),<br>27 January 2011 (27.01.2011),<br>claims 1, 11; paragraphs [0002], [0061]<br>& US 2010/285084 A1     & CA 2704962 A1<br>& EP 2214737 A2       & CN 101909666 A | 6 |
| A | Gu,J.D. et al., Protection of catheter surfaces<br>from adhesion of Pseudomonas aeruginosa by a<br>combination of silver ions and lectins, World<br>J. Microbiol. Biotechnol., 2001.03, Vol.17,<br>No.2, P.173-179 Summary, Fig. 3 | 6 |
| A | Kadam,R.U. et al., A Glycopeptide Dendrimer<br>Inhibitor of the Galactose-Specific Lectin<br>LecA and of Pseudomonas aeruginosa Biofilms,<br>Angew. Chem. Int. Ed., 2011.11.04, Vol.50,<br>No.45, P.10631-10635, page 10631, left column,<br>line 1 to right column, line 4, fig. 1 | 6 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012055939 A
- JP 2007145876 A
- JP 2004155681 A
- JP 2007518400 A
- JP 3849945 B
- JP 2011241192 A